(19) Europäisches Patentamt
European Patent Office
Office européen des brevets

(11) **EP 3 682 818 B1**

(12) **EUROPEAN PATENT SPECIFICATION**

(45) Date of publication and mention
of the grant of the patent:
**08.11.2023 Bulletin 2023/45**

(21) Application number: **18856405.8**

(22) Date of filing: **27.07.2018**

(51) International Patent Classification (IPC):
**A61B 17/12** (2006.01)

(52) Cooperative Patent Classification (CPC):
**A61B 17/12154; A61B 17/12031; A61B 17/12113;**
A61B 17/12145; A61B 2017/00526;
A61B 2017/12068

(86) International application number:
**PCT/JP2018/028266**

(87) International publication number:
**WO 2019/054066 (21.03.2019 Gazette 2019/12)**

(54) **INDWELLING DEVICE FOR EMBOLIZATION**

VERWEILVORRICHTUNG FÜR EMBOLISIERUNG

DISPOSITIF À DEMEURE POUR EMBOLISATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.09.2017 JP 2017174747**

(43) Date of publication of application:
**22.07.2020 Bulletin 2020/30**

(73) Proprietor: **Kaneka Corporation**
**Osaka-shi, Osaka 530-8288 (JP)**

(72) Inventor: **WU, Qian**
**Settsu-shi**
**Osaka 566-0072 (JP)**

(74) Representative: **Hoffmann Eitle**
**Patent- und Rechtsanwälte PartmbB**
**Arabellastraße 30**
**81925 München (DE)**

(56) References cited:
**EP-A1- 2 476 380        WO-A1-2016/140313**
**WO-A1-2016/140314      JP-A- 2004 261 234**

• **WHITE J.B. ET AL: "Coils in a Nutshell: A Review
of Coil Physical Properties", AMERICAN
JOURNAL OF NEURORADIOLOGY, vol. 29, no. 7,
1 August 2008 (2008-08-01) , pages 1242-1246,
XP055798122, US ISSN: 0195-6108, DOI:
10.3174/ajnr.A1067 Retrieved from the Internet:
URL:http://www.ajnr.org/content/ajnr/29/7/
1242.full.pdf>**

**Description**

TECHNICAL FIELD

The present invention relates to an indwelling device for embolization, and particularly to an indwelling device for embolization used for embolization of a lesioned part such as an aneurysm.

BACKGROUND ART

Currently, an endovascular treatment method using a catheter or the like is known as a less invasive treatment method for an intravascular lesion such as an aneurysm. In the endovascular treatment, there is a method of, for example, inserting an indwelling device for embolization (hereinafter simply referred to as an "indwelling device") into an aneurysm through a catheter, placing a part of the indwelling device by cutting off at a detachable portion, and embolizing the aneurysm. A part of the indwelling device placed in the aneurysm becomes a physical obstacle to a blood flow and a thrombus is formed around the part of the indwelling device, whereby a risk of rupture of the aneurysm can be reduced.

An indwelling device has a structure in which a coil portion to be placed in a body and a pusher portion for delivering the coil portion to a predetermined site are connected via a detachable portion. The coil portion is placed at a predetermined site such as an aneurysm, followed by cutting the detachable portion by mechanical, thermal or electrical operation.

The indwelling device is required to have a function so that the coil portion is prevented or suppressed from stretching unlimitedly, in order to reliably perform a repositioning operation that corrects an indwelling state by recovering the coil portion into a catheter after the coil portion pushed out from the catheter is placed at a predetermined site and before cutting.

As a means for preventing or suppressing stretch of the coil portion, there is a method of disposing a stretch-resistant member, which can suppress the stretch of the coil portion in its axial direction, inside the coil portion. The stretch-resistant member is required to have high strength for the reason described above. Accordingly, in order to enhance safety more during the operation, the stretch-resistant member is preferably made of a material having relatively high strength such as, for example, a precious metal and a resin polymer (e.g., polypropylene or polyethylene).

Patent Literature 1 discloses an embolization coil in which an extension-preventing wire and partially or entirely pitch-wound coil are fixed to each other at least two points different from each other. Patent Literature 1 also discloses that the extension-preventing wire has a region having a stretchable shape in a region placed between the two points different from each other. However, there is room for examining the size of the stretchable shape of the extension-preventing wire.

Patent Literature 2 discloses an embolization device for positioning in a blood vessel, comprising an elongated wire body which in its unloaded condition has a predetermined shape and has an elongated shape with a substantially straight center line during its insertion through a catheter to a placement site in the blood vessel and after its release from the catheter assumes a complexly curved shape which depends on the predetermined shape and on the blood vessel impact on the wire body, which wire body preferably has a coupling means at its back end. Patent Literature 2 also discloses a measurement value of a spring constant of the wire body. However, since the embolization device of Patent Literature 2 does not have a stretch-resistant member, the coil portion cannot be prevented from unlimitedly stretching and operability of the coil portion may be likely to be deteriorated.

CITATION LIST

PATENT LITERATURE

PATENT LITERATURE 1
Japanese Unexamined Laid-open Patent Application Publication No. 2015-128677
PATENT LITERATURE 2
Japanese Unexamined Laid-open Patent Application Publication No. 2000-517222 Further related prior art may be found in EP 2 476 380 A1 describing a metal coil made of a platinum-tungsten alloy wire (wire rod external diameter: 45 um) having a coil internal diameter of 150 $\mu$m and a natural length of 200 mm. A pitch-wound region having a gap distance of 30% of the external diameter of the wire rod is formed in the proximal 30 mm region of the coil and a densely wound region in the other region. The rate of the pitch-wound region is 15%. A wire rod (twisted wire) of a platinum-tungsten alloy wire having a natural length of 195 mm in a wave shape at an amplitude of 100 $\mu$m is used as the expandable/shrinkable elongation-preventing wire.

SUMMARY OF INVENTION

The present invention is defined by appended independent claim 1. The dependent claims describe optional features and distinct embodiments.

TECHNICAL PROBLEM

In manufacturing the indwelling device, a sterilization treatment with heating is applied, and at the time, when the stretch-resistant member having a small spring constant is disposed inside the coil portion and sterilized, the size of the coil portion may change. In this case, it becomes difficult to manufacture the indwelling device including the coil portion having an appropriate size. An object of the present invention is to provide an indwelling device for embolization in which a change in size of a coil portion is suppressed at a time when a stretch-resistant member is disposed and sterilized.

SOLUTION TO PROBLEM

As a result of intensive studies for solving the above-mentioned problems, the present inventor has completed the present invention. That is, the present invention and disclosure relates to the following indwelling device for embolization.

[1] An indwelling device for embolization comprising a coil portion having a proximal side and a distal side and having a lumen extending in a longitudinal direction, and a stretch-resistant member disposed in the lumen, wherein

a spring constant of the coil portion is 1.0 N/mm or less,
the stretch-resistant member has a waveform, and
a wave height, which is a distance between peaks on an inner side of the waveform of the stretch-resistant member, is 35 $\mu$m or larger and smaller than an inner diameter of the coil portion.

[2] The indwelling device for embolization according to [1], wherein the spring constant is 0.6 N/mm or less and the wave height is larger than 35 $\mu$m.
[3] The indwelling device for embolization according to [1] or [2], wherein the spring constant is more than 0.2 N/mm.
[4] An indwelling device for embolization comprising a coil portion having a proximal side and a distal side and having a lumen extending in a longitudinal direction, and a stretch-resistant member disposed in the lumen, wherein

a spring constant of the coil portion is 0.2 N/mm or less,
the stretch-resistant member has a waveform, and
a wave height, which is a distance between peaks on an inner side of the waveform of the stretch-resistant member, is 50 $\mu$m or larger and smaller than an inner diameter of the coil portion.

[5] The indwelling device for embolization according to [4], wherein the wave height is larger than 50 $\mu$m.
[6] The indwelling device for embolization according to [4] or [5], wherein the spring constant is more than 0.1 N/mm.
[7] An indwelling device for embolization comprising a coil portion having a proximal side and a distal side and having a lumen extending in a longitudinal direction, and a stretch-resistant member disposed in the lumen, wherein

a spring constant of the coil portion is 0.1 N/mm or less,
the stretch-resistant member has a waveform, and
a wave height, which is a distance between peaks on an inner side of the waveform of the stretch-resistant member, is 65 $\mu$m or larger and smaller than an inner diameter of the coil portion.

[8] The indwelling device for embolization according to [7], wherein the wave height is larger than 65 $\mu$m.
[9] The indwelling device for embolization according to [7] or [8], wherein the spring constant is 0.01 N/mm or more.
[10] The indwelling device for embolization according to any one of [1] to [9], wherein a wavelength of the stretch-resistant member is 280 $\mu$m or longer and 400 $\mu$m or shorter.
[11] The indwelling device for embolization according to any one of [1] to [10], wherein a wire diameter of the coil portion is 0.1 mm or smaller.
[12] The indwelling device for embolization according to any one of [1] to [11], wherein the coil portion, a detachable portion and a pusher portion are disposed in this order from a distal side of the indwelling device, and the coil portion and the pusher portion are connected to each other via the detachable portion.

ADVANTAGEOUS EFFECTS OF INVENTION

According to the indwelling device for embolization of the present invention, a change in size of the coil portion is suppressed at a time when the stretch-resistant member is disposed to the indwelling device and sterilized.

BRIEF DESCRIPTION OF DRAWINGS

Fig. 1 shows an example of a distal part of an indwelling device for embolization of the present invention and represents a partial cross-sectional view seen from a lateral side of the distal part of the indwelling device.
Fig. 2 shows an example of an indwelling device for embolization of the present invention and represents an overall lateral view of the indwelling device.
Fig. 3 shows an example of a stretch-resistant member disposed to the indwelling device for embolization of the present invention and represents a lateral view of the stretch-resistant member.

DESCRIPTION OF EMBODIMENTS

Hereinafter, the present invention will be specifically explained below based on the following embodiments; however, the present invention is not restricted by the embodiments described below of course, and can be certainly put into practice after appropriate modifications within in a range meeting the gist of the above and the below, all of which are included in the technical scope of the present invention. In the drawings, hatching or a reference sign for a member may be omitted for convenience, and in such a case, the description and other drawings should be referred to. In addition, sizes of various members in the drawings may differ from the actual sizes thereof, since priority is given to understanding the features of the present invention.

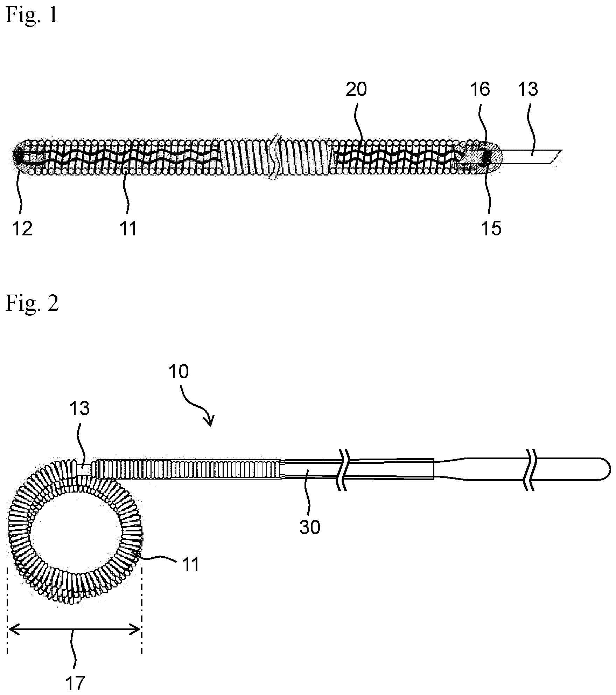

An indwelling device for embolization (hereinafter simply referred to as an "indwelling device") 10 of the present invention comprises a coil portion 11, a detachable portion 13 and a pusher portion 30 from a distal side thereof, as shown in Fig. 2. In the indwelling device 10, a head side of the coil portion 11 is referred to as a distal side, and a side opposite to the detachable portion 13 of the pusher portion 30 is referred to as a proximal side. A distal and proximal direction of the indwelling device 10 is referred to as a longitudinal direction.

The indwelling device 10 comprises the coil portion 11 having a lumen extending in the longitudinal direction. The lumen is a cavity inside the coil. The lumen may extend over the entire length of the coil portion 11 or may have a structure in which a part of the coil lumen is closed. It is preferable that the indwelling device 10 comprises the coil portion 11, and a head part 12 having a substantially hemispherical round shape is disposed at a distal end part of the coil portion 11, that is, a left end part of the coil portion 11 in Fig. 1. The detachable portion 13 having, for example, a rod shape, that connects the coil portion 11 and the pusher portion 30 is disposed at a proximal end part of the coil portion 11. The coil portion 11 and the pusher portion 30 are connected to each other via the detachable portion 13. The detachable portion 13 is disposed so as to protrude and extend proximally from a proximal end of the coil portion 11, that is, so as to protrude and extend toward a right side in Fig. 1, in the state where a part of a distal part thereof is attached to an inner surface of the proximal end part of the coil portion 11. A proximal part of the detachable portion 13 is attached to the pusher portion 30. Therefore, the coil portion 11 is connected to the pusher portion 30 in a detachable state via the part of the detachable portion 13 that protrudes and extends proximally from the proximal end of the coil portion 11, thereby constituting the indwelling device 10. In the indwelling device 10, the shape of the head part 12 is not limited to a substantially hemispherical round shape, and other shapes that do not damage a blood vessel can be adopted.

The coil portion 11 is generally configured by winding a metal wire in a spiral shape. Such a metal wire can be selected from among those that are chemically stable to a human body like noble metals, those that are chemically stabilized by forming a passive film on the surface in vivo, those that have low toxicity or those that are biocompatible when it is left in a human body for a long time. For example, platinum, gold, titanium, tungsten, alloys thereof, stainless steels and the like can be exemplified. Among them, a platinum alloy such as platinum-tungsten is preferably used, since it has chemical stability in vivo, excellent physical properties such as strength and elasticity, and processability.

The coil portion 11 constituting the indwelling device 10 preferably has bendability or flexibility, and preferably has the following configuration, though it may vary depending on the material of the metal wire. A diameter of the metal wire constituting the coil portion 11, that is, a wire diameter is preferably 10 $\mu$m or larger and 120 $\mu$m or smaller. A coil diameter of the coil portion 11 is preferably 100 $\mu$m or larger and 500 $\mu$m or smaller. A coil length of the coil portion 11 is preferably 2 mm or longer and 500 mm or shorter.

The coil portion 11 may have a shape in which a metal wire is wound in a spiral shape, or it may be configured to have a coil shape in which the metal wire is wound in a spiral shape as a primary shape and have a secondary shape in which the primary shape is further formed into a certain shape. The metal wire can be formed into a coil-shaped primary shape by winding around a cylindrical primary mold. Further, the coil portion 11 having the primary shape can be wound around a cylindrical secondary mold to give a coil-shaped secondary shape. Alternatively, the coil portion 11 having the primary shape can be inserted into a predetermined box-shaped secondary mold to give a box-shaped secondary shape. The metal wire, the primary shape or the like can be imparted a certain shape by winding around a mold or inserting into a mold, followed by heating. After imparting the secondary shape to the coil portion 11, a tertiary shape can be further imparted. As the mold, a mold in which a wire or a coil is wound on its outside to impart a shape, or a mold which has a lumen and in which a wire or a coil is inserted to impart a shape according to the lumen, can be used. Fig. 1 shows the coil portion 11 having a primary shape that is linearly extended. This configuration is a form when it is held in a tube such as a catheter. When it is not restrained by a tube wall of a catheter or the like, it exhibits an indefinite shape or a secondary coil shape in which the coil portion 11 is further wound as shown in Fig. 2.

A secondary coil diameter of the coil portion 11 is appropriately determined according to an application site, for example, the size and inner diameter of an aneurysm, and is preferably 1 mm or larger, more preferably 1.5 mm or larger, and preferably 40 mm or smaller, more preferably 20 mm or smaller. The secondary coil diameter of the coil portion 11 means a length indicated by the reference numeral 17 in Fig. 2.

The shape of the detachable portion 13 is a rod shape, and can be a columnar shape, a prismatic shape, or a combination thereof. An outer diameter of the detachable portion 13 varies depending on specific configurations of the pusher portion 30 and the coil portion 11 and is not restricted as long as the target coil portion 11 can be connected to the pusher portion 30 by an appropriate means. The detachable portion 13 preferably has an outer diameter of, for example, 0.05 mm or larger and 2.0 mm or smaller, and preferably has a length of 1.0 mm or longer and 10 mm or shorter.

A material of the detachable portion 13 is preferably those that do not adversely affect a living body and can be deformed or molten by a thermal, mechanical or electrical method, thereby separating the coil portion 11 from the pusher portion 30. Specifically, it is preferable that the detachable portion 13 contains a heat-melt material such as a polyvinyl alcohol polymer that is molten and cut by heating. The material of the detachable portion 13 is not limited to those, and a material which transforms by heating, such as a shape memory alloy and shape memory resin, for example, can be also used. Thereby, the coil portion 11 can be separated from the pusher portion 30 by a thermal operation.

A stretch-resistant member 20 is disposed in the lumen of the coil portion 11. The stretch-resistant member 20 has a linear body, and is disposed so as to be long in the longitudinal direction of the lumen of the coil portion 11. The stretch-resistant member 20 is preferably disposed in the state where the entire stretch-resistant member 20 extends in the longitudinal direction in the lumen of the coil portion 11.

A proximal end part of the stretch-resistant member 20 is preferably anchored to the detachable portion 13. A distal end part of the stretch-resistant member 20 is preferably anchored to a distal part of the coil portion 11 and may be anchored to the head part 12. The stretch-resistant member 20 prevents the coil portion 11 from stretching more than necessary in a catheter or in a living body when the indwelling device 10 passes through the catheter and is delivered into the body.

The stretch-resistant member 20 has a waveform. That is, as viewed from a lateral side of the indwelling device 10, the stretch-resistant member 20 is formed in a waveform. Examples of the waveform include a substantially sine wave shape, a substantially rectangular wave shape, and a substantially triangular wave shape, and among them, a substantially sine wave shape is preferable. The stretch-resistant member 20 is preferably imparted a substantially sine wave shape over the entire distal and proximal direction, as shown in Fig. 1. The coil portion 11 is extendable in a range until the stretch-resistant member 20 is tensioned, that is, in a range until the stretch-resistant member 20 is elongated in a straight linear fashion. The stretch-resistant member 20 is preferably held in a contracted state, that is, in a state where the waveform is imparted inside the coil portion 11, until the coil portion 11 is placed in a tumor. In addition, a natural length of the stretch-resistant member 20, that is, the length where the stretch-resistant member 20 is elongated linearly, is preferably longer than a natural length of the coil portion 11, that is, the length of the primary coil of the coil portion 11. Details of the size of the waveform of the stretch-resistant member 20 are described below.

The stretch-resistant member 20 can be composed of a single monofiber or a stranded wire thereof. Alternatively, the stretch-resistant member 20 can be composed of, for example, a metal wire of a platinum alloy such as platinum-tungsten in the same manner as the coil portion 11. In the case where the stretch-resistant member 20 is composed of a metal wire, there is a possibility that the stretch-resistant member 20 may be ruptured by metal fatigue when the coil portion 11 is repeatedly put in and out of a catheter in placing the coil portion 11 at an appropriate site in a body. In order to prevent this, the stretch-resistant member 20 can be composed of, for example, a metal material which is resistant to metal fatigue or a resin material which does not cause metal fatigue.

Examples of a constituent material of a resin wire that can be used for the stretch-resistant member 20 include synthetic resins such as polyethylene, polypropylene, polyethylene terephthalate, polyamide, polyester, polylactic acid, polyglycolic acid, poly (lactic acid-glycolic acid) copolymer, polyhydroxybutyric acid, polyhydroxybutyrate valeric acid and a copolyester of 3-hydroxybutyric acid and 3-hydroxyhexanoic acid copolyester; polymers derived from a biodegradable polymer such as cellulose, polydioxanone, protein and vinyl polymer; and others. Among them, from the viewpoint of biocompatibility, the resin wire of the stretch-resistant member 20 is preferably made of polyethylene, polypropylene, nylon, polyester, polydioxanone, polytetrafluoroethylene, polyglycolic acid, polylactic acid, silk, or a composite material composed by any combination thereof.

As a method for anchoring the distal end part of the stretch-resistant member 20 to the distal part of the coil portion 11 or the head part 12 or a method for anchoring the proximal end part of the stretch-resistant member 20 to the detachable portion 13, adhesion using an adhesive, welding by including fusion or a inclusion, crimping such as mechanical caulking, physical connection, ligation, or other methods can be used, for example. Anchoring of the stretch-resistant member 20 to the distal part of the coil portion 11 may be performed by, for example, disposing a structure formed by winding one round in a small spiral shape at the head part 12 of the coil portion 11 and hanging the stretch-resistant member 20 on this structure. In this case, both ends of the stretch-resistant member 20 may be anchored to the rod-shaped detachable portion 13.

The indwelling device 10 is sterilized with ethylene oxide gas after assembly. Since the sterilization treatment is accompanied by heating, the stretch-resistant member 20 made of a resin wire is heated in a contracted state, and may be solidified while maintaining the contracted shape after cooling. As a result, the natural length of the stretch-resistant member 20 after sterilization may become shorter than that before sterilization, and the secondary coil diameter of the coil portion 11 may change. The softer the coil portion 11 is, the greater the influence that the secondary coil diameter is affected by shortening of the stretch-resistant member 20 is. If the secondary coil diameter of the coil portion 11 changes greatly after disposing the stretch-resistant member 20 in the coil portion and sterilizing, it becomes difficult to adjust the secondary coil diameter of the coil portion 11 to a desired size in the indwelling device 10 after sterilization. In this case, it becomes difficult to manufacture the indwelling device 10 comprising the coil portion 11 having an appropriate specification suitable for the size of an aneurysm or the like.

Softness of the coil portion 11 can be defined by a spring constant k of the coil. The spring constant k is expressed by the following equation, $D_1$ represents the wire diameter of the coil portion 11, G represents a shear modulus specific to the material of the coil portion 11, $D_2$ represents the primary coil diameter of the coil portion 11, and n represents a number of loops of the wire per 1 mm of the coil portion 11, that is, a number of loops of a metal wire. The coil portion 11 is assumed to be formed by densely winding a metal wire in a spiral shape; and in this case, the number n of loops of a wire per 1 mm of the coil portion 11 is the reciprocal of the wire diameter. As the value of the spring constant k is smaller, the coil portion 11 is softer and more susceptible to the shortening of the stretch-resistant member 20 after the sterilization treatment.

[Equation 1]

$$k = \frac{D_1{}^4 G}{8 D_2{}^3 n}$$

The coil portion 11 preferably has a spring constant of 1.0 N/mm or less, and more preferably 0.6 N/mm or less. Thereby, breaking through an aneurysm due to the coil portion 11 being too hard is less occurred. From the same viewpoint, the wire diameter of the coil portion 11 is preferably 0.1 mm or smaller, and thereby, the coil portion 11 is prevented from being too hard. Meanwhile, the lower limit value of the spring constant of the coil portion 11 is preferably 0.01 N/mm or more, from the viewpoint of ensuring a shape retaining property of the coil portion 11.

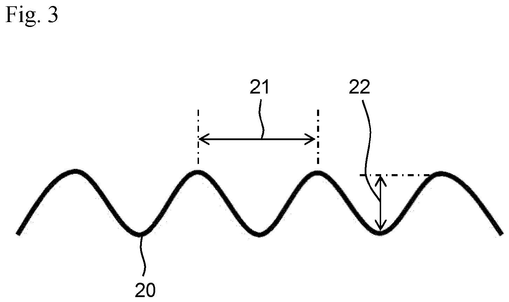

As shown in Fig. 3, it is preferable that the distance between crests of the resin wire, namely a wavelength 21, of the stretch-resistant member 20 to which the waveform is imparted is 280 $\mu$m or longer and 400 $\mu$m or shorter. More preferably, the average of the wavelength of the stretch-resistant member 20 to which the waveform is imparted is in such a range. As a result, it becomes easy to make the entire length of the stretch-resistant member 20 longer than the length of the coil portion 11, that provides a margin for stretching when the coil portion 11 is placed in a predetermined site such as an aneurysm.

It is preferable that the stretch-resistant member 20 has a predetermined wave height. As shown in Fig. 3, the wave height 22 means a distance between peaks on the inner side of the waveform of the stretch-resistant member 20, that is, the distance between a crest and a trough of the resin wire to which the waveform is imparted, excluding the wire diameter of the resin wire. A preferred dimension of the wave height 22 varies depending on the spring constant of the coil portion 11, and is preferably, for example, 35 $\mu$m or larger, or larger than 35 $\mu$m. More preferably, the average of the wave height of the stretch-resistant member 20 is in such a range. The upper limit value of the wave height of the stretch-resistant member 20 is not particularly limited as long as it is smaller than the inner diameter of the coil portion 11. As the wave height of the waveform applied to the stretch-resistant member 20 increases, the change rate of the secondary coil diameter of the coil portion 11 decreases between before and after the sterilization treatment, and it becomes easy to adjust the secondary coil diameter of the coil portion 11 to a desired size after the stretch-resistant member 20 is disposed in the lumen of the coil portion 11 and subjected to the sterilization treatment.

In the stretch-resistant member 20, it is preferable that the smaller the spring constant of the coil portion 11 is, the larger the wave height is. Thereby, even when the wave height of the stretch-resistant member 20 becomes smaller after the sterilization treatment, the influence on the coil portion 11 which has a small spring constant and is made soft can be suppressed to the minimum.

For example, in the case where the spring constant of the coil portion 11 is 1.0 N/mm or less, the wave height of the stretch-resistant member 20 is preferably 35 $\mu$m or larger and smaller than the inner diameter of the coil portion 11. The wave height of the stretch-resistant member 20 may be larger than 35 $\mu$m. In this case, the spring constant of the coil portion 11 is preferably 0.6 N/mm or less and preferably more than 0.2 N/mm.

In the case where the spring constant of the coil portion 11 is 0.2 N/mm or less, the wave height of the stretch-resistant member 20 is preferably 50 $\mu$m or larger and smaller than the inner diameter of the coil portion 11. The wave height of the stretch-resistant member 20 may be larger than 50 $\mu$m. In this case, the spring constant of the coil portion 11 is preferably more than 0.1 N/mm.

In the case where the spring constant of the coil portion 11 is 0.1 N/mm or less, the wave height of the stretch-resistant member 20 is preferably 65 $\mu$m or larger and smaller than the inner diameter of the coil portion 11. The wave height of the stretch-resistant member 20 may be larger than 65 $\mu$m. In this case, the spring constant of the coil portion 11 is preferably 0.01 N/mm or more.

EXAMPLES

Hereinafter, examples performed for confirming effects of the indwelling device of the present invention are explained.

(Preparation Example 1)

Using a platinum-tungsten alloy wire having a wire diameter of 0.05 mm, a coil portion of a primary shape having a coil diameter of 0.35 mm was prepared. The obtained coil portion was wound around a cylindrical secondary mold having a predetermined diameter and heated to give a secondary shape to the coil portion. A stretch-resistant member was prepared by imparting a substantially sine wave shape having a wave height of 35 $\mu$m and a wavelength of 340 $\mu$m to a polypropylene resin wire and folding back, and the resultant was disposed inside the coil portion. The stretch-resistant member was arranged such that the folded portion of the resin wire was hanged on a distal end of the coil portion and both ends of the resin wire extended out from a proximal end of the coil portion, and a rod-shaped detachable portion was inserted into the proximal end of the coil portion. An adhesive was injected into a distal end part and a proximal end part of the coil portion, respectively, and the stretch-resistant member was fixed to the distal end part and the proximal end part of the coil portion. A proximal end part of the detachable portion, that extended proximally from the proximal end of the coil portion, was plugged into a pusher portion, and the detachable portion was attached to the pusher portion with an adhesive, thereby assembling an indwelling device. Thus obtained indwelling device was placed into the interior of a spiral tube and sterilized. The resultant was referred to as an "indwelling device 1-1".

(Preparation Example 2)

An indwelling device was assembled and sterilized in the same manner as Preparation Example 1, except that a resin wire to which a substantially sine wave shape having a wave height of 50 $\mu$m and a wavelength of 340 $\mu$m was imparted was used for the resin wire constituting the stretch-resistant member. The resultant was referred to as an "indwelling device 1-2".

(Preparation Example 3)

An indwelling device was assembled and sterilized in the same manner as Preparation Example 1, except that a secondary mold having a smaller diameter was used as the cylindrical secondary mold for imparting a secondary shape to the coil portion and a resin wire to which a substantially sine wave shape having a wave height of 65 $\mu$m and a wavelength of 340 $\mu$m was imparted was used for the resin wire constituting the stretch-resistant member. The resultant was referred to as an "indwelling device 1-3".

(Preparation Example 4)

An indwelling device was assembled and sterilized in the same manner as Preparation Example 1, except that the coil portion was formed from a platinum-tungsten alloy wire having a strand wire diameter of 0.06 mm. The resultant was referred to as an "indwelling device 2-1".

(Preparation Example 5)

An indwelling device was assembled and sterilized in the same manner as Preparation Example 4, except that a resin wire to which a substantially sine wave shape having a wave height of 50 $\mu$m and a wavelength of 340 $\mu$m was imparted was used for the resin wire constituting the stretch-resistant member. The resultant was referred to as an "indwelling device 2-2".

(Preparation Example 6)

An indwelling device was assembled and sterilized in the same manner as Preparation Example 4, except that a resin wire to which a substantially sine wave shape having a wave height of 65 $\mu$m and a wavelength of 340 $\mu$m was imparted was used for the resin wire constituting the stretch-resistant member. The resultant was referred to as an "indwelling device 2-3".

(Preparation Example 7)

An indwelling device was assembled and sterilized in the same manner as Preparation Example 4, except that a secondary mold having a larger diameter was used as the cylindrical secondary mold for imparting a secondary shape to the coil portion and a resin wire to which a substantially sine wave shape having a wave height of 50 $\mu$m and a wavelength of 340 $\mu$m was imparted was used for the resin wire constituting the stretch-resistant member. The resultant was s referred to as an "indwelling device 2-4".

(Preparation Example 8)

An indwelling device was assembled and sterilized in the same manner as Preparation Example 1, except that a secondary mold having a larger diameter was used as the cylindrical secondary mold for imparting a secondary shape to the coil portion, the coil portion was formed from a platinum-tungsten alloy wire having a strand wire diameter of 0.07 mm, and a resin wire to which a wave shape was not imparted was used for the resin wire constituting the stretch-resistant member. The resultant was referred to as an "indwelling device 3-1".

(Preparation Example 9)

An indwelling device was assembled and sterilized in the same manner as Preparation Example 8, except that a resin wire to which a substantially sine wave shape having a wave height of 35 $\mu$m and a wavelength of 340 $\mu$m was imparted was used for the resin wire constituting the stretch-resistant member. The resultant was referred to as an "indwelling device 3-2".

For each indwelling device prepared as described above, the secondary coil diameters of the coil portion before installing the stretch-resistant member and after the sterilization treatment were measured. The secondary coil diameter of the coil portion was determined by placing the coil portion or the indwelling device in an unloaded state under an ordinary temperature environment to form a secondary coil shape which the coil portion of the primary shape was wound (the winding number was 3) as shown in Fig. 2, and measuring using a standard type vernier caliper (manufactured by Niigata Seiki Co., Ltd.). The results are shown in Table 1 below. In Table 1, the secondary coil diameter change rate was calculated by the following formula: secondary coil

diameter change rate (%) = (|secondary coil diameter before placement of the stretch-resistant member − secondary coil diameter after sterilization| / secondary coil diameter before placement of the stretch-resistant member) × 100.

[TABLE 1]

| | Indwelling device 1-1 | Indwelling device 1-2 | Indwelling device 1-3 | Indwelling device 2-1 | Indwelling device 2-2 | Indwelling device 2-3 | Indwelling device 2-4 | Indwelling device 3-1 | Indwelling device 3-2 |
|---|---|---|---|---|---|---|---|---|---|
| Wire diameter: $D_1$ (mm) | 0.05 | | | 0.06 | | | | 0.07 | |
| Primary coil diameter: $D_2$ (mm) | 0.35 | | | 0.35 | | | | 0.36 | |
| Number of loops of wire per 1 mm: n | 20 | | | 17 | | | | 14 | |
| Shear modulus of Pt: G (GPa) | 61 | | | 61 | | | | 61 | |
| Spring constant: k (N/mm) | 0.06 | | | 0.14 | | | | 0.27 | |
| Wave height of stretch-resistant member ($\mu$m) | 35 | 50 | 65 | 35 | 50 | 65 | 50 | 0 | 35 |
| Wavelength of stretch-resistant member ($\mu$m) | 340 | 340 | 340 | 340 | 340 | 340 | 340 | - | 340 |
| Secondary coil diameter before placement of stretch-resistant member (mm) | 5.7 | 5.8 | 2.1 | 5.8 | 5.8 | 5.8 | 12.1 | 12.0 | 12.1 |
| Secondary coil diameter after sterilization (mm) | 6.7 | 6.4 | 2.1 | 6.6 | 6.0 | 5.9 | 12.1 | 13.3 | 12.0 |
| Secondary coil diameter change rate | 18% | 10% | 2% | 14% | 4% | 2% | 0% | 10% | 1% |

In the indwelling devices 1-1 to 1-3, stretch-resistant members having different wave heights to each other were disposed inside a coil portion having a spring constant k of 0.06 N/mm, that was 0.1 N/mm or less. Provided that the secondary coil diameter change rate of the coil portion was 5% or less was a non-defective product, the indwelling device 1-3 in which the stretch-resistant member had a wave height of 65 $\mu$m was resulted in having a secondary coil diameter change rate of 2%, that was 5% or less, and could be classified as a non-defective product. Therefore, for a coil portion having a spring constant k of 0.1 N/mm or less, the wave height of the stretch-resistant member is preferably 65 $\mu$m or larger.

In the indwelling devices 2-1 to 2-4, stretch-resistant members having different wave heights to each other were disposed inside a coil portion having a spring constant k of 0.14 N/mm, that was 0.2 N/mm or less. Provided that the secondary coil diameter change rate of the coil portion was 5% or less was a non-defective product, the coils in which the stretch-resistant member had a wave height of 50 $\mu$m or 65 $\mu$m were resulted in having a secondary coil diameter change rate of 4%, 2% or 0%, that were 5% or less, and could be classified as non-defective products. Therefore, for a coil portion having a spring constant k of 0.2 N/mm or less, the wave height of the stretch-resistant member is preferably 50 $\mu$m or larger.

In the indwelling devices 3-1 and 3-2, stretch-resistant members having different wave heights to each other were disposed inside a coil portion having a spring constant k of 0.27 N/mm, that was 1.0 N/mm or less. Provided that the secondary coil diameter change rate of the coil portion was 5% or less was a non-defective product, the coil in which the stretch-resistant member had a wave height of 35 $\mu$m was resulted in having a secondary coil diameter change rate of 1%, that was 5% or less, and could be classified as a non-defective product. Therefore, for a coil portion having a spring constant k of 1.0 N/mm or less, the wave height of the stretch-resistant member is preferably 35 $\mu$m or larger.

From the above results, the indwelling devices 1-3, 2-2 to 2-4, and 3-2 had the secondary coil diameter change rate of the coil portion of 5% or less and change in the secondary coil diameter of the coil portion after sterilization was small. As a result, it was confirmed that the indwelling device for embolization with a small dimensional change of the coil portion after placement of the stretch-resistant member can be provided.

REFERENCE SIGNS LIST

10: an indwelling device for embolization
11: a coil portion
12: a head part
13: a detachable portion
16: an adhesive
17: a secondary coil diameter
20: a stretch-resistant member
21: a wavelength
22: a wave height
30: a pusher portion

**Claims**

1. An indwelling device for embolization (10) comprising a coil portion (11) having a proximal side and a distal side and having a lumen extending in a longitudinal direction, and a stretch-resistant member (20) disposed in the lumen, wherein

   the stretch-resistant member has a waveform, and
   a wave height (22), which is a distance between peaks on an inner side of the waveform of the stretch-resistant member, is 35 $\mu$m or larger and smaller than an inner diameter of the coil portion, **characterised in that** a spring constant of the coil portion is 1.0 N/mm or less.

2. The indwelling device for embolization according to claim 1, wherein the spring constant is 0.6 N/mm or less and the wave height is larger than 35 $\mu$m.

3. The indwelling device for embolization according to claim 1 or 2, wherein the spring constant is more than 0.2 N/mm.

4. The indwelling device for embolization according to claim 1, wherein

the spring constant of the coil portion is 0.2 N/mm or less, and
the wave height is 50 $\mu$m or larger and smaller than an inner diameter of the coil portion.

5. The indwelling device for embolization according to claim 4, wherein the wave height is larger than 50 $\mu$m.

6. The indwelling device for embolization according to claim 4 or 5, wherein the spring constant is more than 0.1 N/mm.

7. The indwelling device for embolization according to claim 1, wherein

the spring constant of the coil portion is 0.1 N/mm or less, and
the wave height is 65 $\mu$m or larger and smaller than an inner diameter of the coil portion.

8. The indwelling device for embolization according to claim 7, wherein the wave height is larger than 65 $\mu$m.

9. The indwelling device for embolization according to claim 7 or 8, wherein the spring constant is 0.01 N/mm or more.

10. The indwelling device for embolization according to any one of claims 1 to 9, wherein a wavelength (21) of the stretch-resistant member is 280 $\mu$m or longer and 400 $\mu$m or shorter.

11. The indwelling device for embolization according to any one of claims 1 to 10, wherein a wire diameter of the coil portion is 0.1 mm or smaller.

12. The indwelling device for embolization according to any one of claims 1 to 11, wherein

the coil portion, a detachable portion (13) and a pusher portion (30) are disposed in this order from a distal side of the indwelling device, and
the coil portion and the pusher portion are connected to each other via the detachable portion.


**Patentansprüche**

1. Verweilvorrichtung zur Embolisation (10), umfassend einen Spiralabschnitt (11) mit einer proximalen Seite und einer distalen Seite und mit einem Lumen, das sich in einer Längsrichtung erstreckt, und ein dehnungsresistentes Element (20), das in dem Lumen angeordnet ist, wobei

das dehnungsresistente Element eine Wellenform aufweist, und
eine Wellenhöhe (22), die ein Abstand zwischen Scheitelpunkten auf einer Innenseite der Wellenform des dehnungsresistenten Elements ist, 35 $\mu$m oder größer und kleiner als ein Innendurchmesser des Spiralabschnitts ist, **dadurch gekennzeichnet, dass** eine Federkonstante des Spiralabschnitts 1,0 N/mm oder weniger beträgt.

2. Verweilvorrichtung zur Embolisation nach Anspruch 1, wobei die Federkonstante 0,6 N/mm oder weniger beträgt und die Wellenhöhe größer als 35 $\mu$m ist.

3. Verweilvorrichtung zur Embolisation nach Anspruch 1 oder 2, wobei die Federkonstante mehr als 0,2 N/mm beträgt.

4. Verweilvorrichtung zur Embolisation nach Anspruch 1, wobei

die Federkonstante des Spiralabschnitts 0,2 N/mm oder weniger beträgt, und
die Wellenhöhe 50 $\mu$m oder größer und kleiner als der Innendurchmesser des Spiralabschnitts ist.

5. Verweilvorrichtung zur Embolisation nach Anspruch 4, wobei die Wellenhöhe größer als 50 $\mu$m ist.

6. Verweilvorrichtung zur Embolisation nach Anspruch 4 oder 5, wobei die Federkonstante mehr als 0,1 N/mm beträgt.

7. Verweilvorrichtung zur Embolisation nach Anspruch 1, wobei

die Federkonstante des Spiralabschnitts 0,1 N/mm oder weniger beträgt, und

die Wellenhöhe 65 μm oder größer und kleiner als der Innendurchmesser des Spiralabschnitts ist.

8. Verweilvorrichtung zur Embolisation nach Anspruch 7, wobei die Wellenhöhe größer als 65 μm ist.

9. Verweilvorrichtung zur Embolisation nach Anspruch 7 oder 8, wobei die Federkonstante 0,01 N/mm oder mehr beträgt.

10. Verweilvorrichtung zur Embolisation nach einem der Ansprüche 1 bis 9, wobei eine Wellenlänge (21) des dehnungsresistenten Elements 280 μm oder länger und 400 μm oder kürzer ist.

11. Verweilvorrichtung zur Embolisation nach einem der Ansprüche 1 bis 10, wobei ein Drahtdurchmesser des Spiralabschnitts 0,1 mm oder kleiner ist.

12. Verweilvorrichtung zur Embolisation nach einem der Ansprüche 1 bis 11, wobei

der Spiralabschnitt, ein ablösbarer Abschnitt (13) und ein Schieberabschnitt (30) in dieser Reihenfolge von einer distalen Seite der Verweilvorrichtung aus angeordnet sind, und
der Spiralabschnitt und der Schieberabschnitt über den ablösbaren Teil miteinander verbunden sind.

## Revendications

1. Dispositif à demeure pour embolisation (10) comprenant une partie (11) bobine présentant un côté proximal et un côté distal et ayant une lumière s'étendant dans une direction longitudinale, et un élément (20) résistant à l'étirement disposé dans la lumière, dans lequel

l'élément résistant à l'étirement présente une forme d'onde, et
une hauteur (22) d'onde, qui est une distance entre des pics sur un côté interne de la forme d'onde de l'élément résistant à l'étirement, est de 35 μm ou plus et inférieure à un diamètre interne de la partie bobine, **caractérisé en ce qu'**une constante de ressort de la partie bobine est de 1,0 N/mm ou moins.

2. Dispositif à demeure pour embolisation selon la revendication 1, dans lequel la constante de ressort est de 0,6 N/mm ou moins et la hauteur d'onde est supérieure à 35 μm.

3. Dispositif à demeure pour embolisation selon la revendication 1 ou la revendication 2, dans lequel la constante de ressort est supérieure à 0,2 N/mm.

4. Dispositif à demeure pour embolisation selon la revendication 1, dans lequel

la constante de ressort de la partie bobine est de 0,2 N/mm ou moins, et
la hauteur d'onde est de 50 μm ou plus et inférieure à un diamètre interne de la partie bobine.

5. Dispositif à demeure pour embolisation selon la revendication 4, dans lequel la hauteur d'onde est supérieure à 50 μm.

6. Dispositif à demeure pour embolisation selon la revendication 4 ou la revendication 5, dans lequel la constante de ressort est supérieure à 0,1 N/mm.

7. Dispositif à demeure pour embolisation selon la revendication 1, dans lequel

la constante de ressort de la partie bobine est de 0,1 N/mm ou moins, et
la hauteur d'onde est de 65 μm ou plus et inférieure à un diamètre interne de la partie bobine.

8. Dispositif à demeure pour embolisation selon la revendication 7, dans lequel la hauteur d'onde est supérieure à 65 μm.

9. Dispositif à demeure pour embolisation selon la revendication 7 ou la revendication 8, dans lequel la constante de ressort est de 0,01 N/mm ou plus.

10. Dispositif à demeure pour embolisation selon l'une quelconque des revendications 1 à 9, dans lequel une longueur d'onde (21) de l'élément résistant à l'étirement est de 280 μm ou plus et de 400 μm ou moins.

11. Dispositif à demeure pour embolisation selon l'une quelconque des revendications 1 à 10, dans lequel un diamètre de fil métallique de la partie bobine est de 0,1 mm ou moins.

12. Dispositif à demeure pour embolisation selon l'une quelconque des revendications 1 à 11, dans lequel

la partie bobine, une partie (13) détachable et une partie (30) poussoir sont disposées dans cet ordre à partir d'un côté distal du dispositif à demeure, et
la partie bobine et la partie poussoir sont raccordées l'une à l'autre via la partie détachable.

Fig. 1

Fig. 2

Fig. 3

**EP 3 682 818 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015128677 A
- JP 2000517222 A
- EP 2476380 A1